# EUROPEAN PATENT SPECIFICATION

(11) **EP 1 413 308 B1**
(45) Date of publication and mention of the grant of the patent: **20.06.2007**
(21) Application number: 02746163.1
(22) Date of filing: 02.08.2002
(51) Int. Cl.: A61K 31/575, A61P 3/10, C07J 9/00

(54) **HYPOGLYCEMIC AGENT**
HYPOGLYKÄMISCHES MITTEL
AGENT HYPOGLYCEMIANT

(30) Priority: 03.08.2001 JP 2001235747
(43) Date of publication of application: 28.04.2004
(73) Proprietor: RIKEN, Wako-shi, Saitama 351-0198 (JP); Japan Science and Technology Corporation, Kawaguchi-shi, Saitama 332-0012 (JP); Suzuki, Kunio, Tsurugashima-shi, Saitama 350-2202 (JP)
(72) Inventor: SUZUKI, Kunio, Tsurugashima-shi, Saitama 350-2202 (JP); HIRATA, Kohji, RIKEN, Wako-shi, Sa itama 351-0198 (JP); KONNO, Rie, RIKEN, Wako-shi, Saita ma 351-0198 (JP); MORIIZUMI, Miwa, RIKEN, Wako-shi, Sai tama 351-0198 (JP)
(74) Representative: polypatent
(86) International application number: PCT/JP2002/007906
(87) International publication number: WO 2003/013546

(56) References cited:
- EP-A1- 0 916 342
- WO-A-01/05807
- FOOD CHEMISTRY vol. 54, no. 1, 1995, pages 9 - 13, XP002957383
- HORMONE AND METABOLIC RESEARCH vol. 34, no. 3, 2002, pages 121 - 126, XP002957384
- CHEMICAL ABSTRACTS, vol. 121, Columbus, Ohio, US; abstract no. 292369, XP002957385

## Description

### Technical Field

The present invention relates to a hypoglycemic agent which is useful for therapeutic treatment of diabetes.

### Background Art

Diabetes is a metabolic abnormality of sugars, proteins, and lipids resulting from insufficient secretion and activity of insulin. A patient with the disease will develop symptoms such as polyposia, polyuria, and weight loss, and if the disease is prolonged chronically, sever dysfunctions such as retinopathy, nephropathy, neuropathy, myocardial infarction, and cerebral infarction will arise. The number of patients with diabetes tends to be increasing all over the world. In our country, the number reached to millions in the 1990s, and a ratio has become as much as 5 to 10% of adults over the age of 40. Among the patients, patients with Type-1 (insulin dependent) diabetes are not more than 3% based on the total patient, which diabetes is frequently caused in 25 or less-year-old youths due to extreme deficiency or cease of insulin secretion. Most of the patients are those with Type-2 (non insulin dependent) diabetes resulting from insufficient secretion and activity of insulin.

The cause of type-2 diabetes is not attributable to a single factor. It is considered that relative deficiency of insulin and lowering of its potency (insulin resistance) are generated by an inherited predisposition and additionally by environmental factors such as overeating, obesity, and underexercise, and as a result, hyperglycemia, ketosis, and hyperlipidemia are caused to develop diabetes. Hyperglycemia is not only a resultant symptom but a cause of further aggravation of diabetes (glucose toxicity). Further, on the basis of recent studies, an increase of free fatty acid in blood is also considered as a cause of an aggravation (fat toxicity). However, recent investigation in our country revealed that a ratio of Type-2 diabetes which accompanies no obesity or hyperlipidemia is about 30%, and a true etiogenic mechanism has not yet been clarified (Ryo-ya Ueda et al., Himan-Kenkyu (Journal of Japan Society for the Study of Obesity), Vol. 6(No.3), pp.4-7, 2000).

For treatment of Type-2 diabetes, insulin preparations and oral hypoglycemic agents are used. Among them, insulin preparations are used for patients with complications of hyperglycemia or ketosis. However, the preparations are administered by injections, and therefore, determinations of doses and frequency of administration are difficult. Examples of the oral hypoglycemic agents generally used for treatment of Type-2 diabetes include sulfonylurea-type agents, thiazolidine derivative-type agents, biguanide-type agents, and a -glucosidase inhibitors. Among them, the sulfonylurea-type agents have a risk of causing hypoglycemia, the thiazolidine derivative-type agents have a risk of hepatic disorder, the biguanide-type agents have a risk of lactic acidosis, and the α-glucosidase inhibitors often causes side effects on the digestive tract. Further, these agents are directed to lowering of blood glucose, and these agents, except the thiazolidine derivative-type agents, have no lowering effect on free fatty acid in blood which is considered as another cause of the disease. Accordingly, these available agents are not satisfactory medicaments from viewpoints of a route of administration, side effects, and efficacy, and a medicament has been desired which has a reduced side effect and is safe and easily administered.

24-Alkylcholestan-3-ones and 24-alkylcholesten-3-ones are known to be useful as antiobesity agents and agents for improvement of lipid metabolism (Japanese Patent Unexamined Publication (KOKAI) No. (Hei)11-193296). The above publication discloses that the aforementioned compounds can be applied to diseases with abnormal lipid metabolism, and that the diseases with abnormal lipid metabolism includes arteriosclerosis, hypertension, diabetes, and gout, as well as hyperlipidemia or fatty liver resulting from the abnormality of lipid metabolism (column 16 of the patent publication). However, the above publication neither teaches nor suggests that the aforementioned compounds have hypoglycemic action.

### Disclosure of Invention

An object of the present invention is to provide a highly safe hypoglycemic agent useful for treatment of diabetes. In particular, the object of the present invention is to provide a hypoglycemic agent that successfully exerts hypoglycemic action in Type-2 diabetes which does not always accompany obesity and abnormality of lipid metabolism. The inventors of the present invention conducted various studies to achieve the foregoing object, and as a result, they found that 24-Alkylcholestan-3-ones and 24-alkylcholesten-3-ones had remarkable hypoglycemic action and the compound were free from side effects such as hypoglycemia or diarrhea. The present invention was achieved on the basis of these findings.

The present invention thus provides a hypoglycemic agent wherein the compound is 5-campesten-3-one. The hypoglycemic agent of the present invention can be used for improvement of hyperglycemia in diabetes, and preferably, the agent can be used for improvement of hyperglycemia in Type-2 diabetes. The hypoglycemic agent of the present invention also has a lowering effect on urosaccharide, and accordingly, the agent can be used to lower urinary saccharide as well as to lower blood sugar.

### Brief Explanation of Drawings

Fig. 1 shows changes of a blood sugar level of each group of animals in test example.
Fig. 2 shows results of oral glucose tolerance test (OGTT) applied to each group of animals (19-week old) in test example.
Fig. 3 shows body weights of each group of animals in test example.

### Best Mode for Carrying out the Invention

The active ingredient of the hypoglycemic agent of the present invention is 5-campesten-3-one.

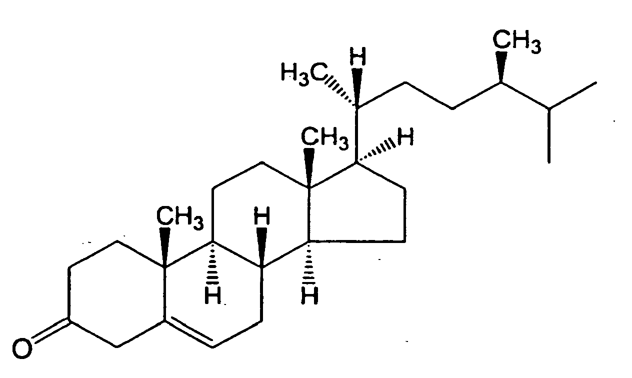

24-Alkylcholestan-3-ones and 24-alkylcholesten-3-ones can be readily prepared by those skilled in the art according to known methods for preparation of cholestan-3-one, cholesten-3-one and the like. For example, the preparation of 5-sitosten-3-one, a typical compound that falls within the compounds represented by the formula (I), is specifically explained in the paper by Parish (Parish, E.J., et al., Synthetic Communications, 22(19), pp.2839-2847, 1992): The compound can also be readily prepared by the method of Cheng (Cheng, Y.S., et al., Synthesis 1980, 223) by using β -sitosterol as a starting material. 5-Campesten-3-one can be synthesized from campesterol according to the method of Shimizu et al. (Japanese Patent Unexamined Publication (KOKAI) No. (Hei)11-295889). Accordingly, it can be readily understood by those skilled in the art that 24-alkylcholestan-3-ones and 24-alkylcholesten-3-ones can be prepared according to the methods described in the above publications, or by referring to these methods and appropriately modifying or altering reaction conditions, reagents and the like, if required. Methods for preparing 24-alkylcholestan-3-ones and 24-alkylcholesten-3-ones, preferably the compounds represented by the formula (I), are not limited to chemical synthetic method, and they can be prepared by biological processes, e.g., culturing microorganisms and the like, or by using enzymes deriving from microorganisms.

The hypoglycemic agent of the present invention can be used as agent for reducing blood sugar. The hypoglycemic agent of the present invention can be applied to pathological conditions such as diabetes wherein hyperglycemia is observed, and the agent has an action of rapidly improving hyperglycemic conditions to reduce blood sugar to a normal level. Causes of hyperglycemic pathological conditions, to which the hypoglycemic agents of the present invention are applicable, are not particularly limited. Examples of the causes of hyperglycemia include diabetes, as well as side effects of medicaments (for example, adrenocortical hormones, β-blockers, hypotensive diuretics, calcium antagonists and the like), pancreatic failures, chromic hepatic diseases, endocrine diseases, intracranial hypertensions, obesity, bulimia, alcohol crapula, alimentary hyperglycemia after gastrectomy, pyretogenous diseases such as infectious diseases, carbon monoxide poisoning, and acute stresses (such as cardiac infarction, cerebral thrombosis and the like). A preferred example of a disease to be applicable by the hypoglycemic agents of the present invention includes diabetes-induced hyperglycemic pathological condition. A particularly preferred example of an applicable disease includes Type-2 diabetes-induced hyperglycemia.

As the hypoglycemic agent of the present invention, the compound, per se, may be used. However, it is generally preferred to use a pharmaceutical composition which is prepared by using pharmaceutically acceptable additives. The hypoglycemic agent of the present invention can be administered orally or parenterally. Examples of the pharmaceutical compositions suitable for oral administrations include, for example, tablets, granules, capsules, powders, solutions, suspensions, syrups and the like. Examples of the pharmaceutical compositions suitable for parenteral administrations include, for example, injections, drip infusions, suppositories, transdermal preparations and the like. However, dosage forms of the hypoglycemic agents of the present invention are not limited to these examples. Doses of the hypoglycemic agent of the present invention should be appropriately increased or decreased depending on the age, conditions and the like of a patient, the route of administration and other factors. Generally, the dose may be within the range of 1 to 5,000 mg per day for an adult.

Pharmaceutical additives used for the preparation of the hypoglycemic agent of the present invention are not particularly limited, and they can be suitably chosen by those skilled in the art. For example, excipients, binders, lubricants, dispersing agents, suspending agents, emulsifiers, buffering agents, antioxidants, antiseptics, isotonic agents, pH modifiers, solubilizing agents, stabilizers and the like can be used. Substances used for each of the aforementioned purposes are well known to those skilled in the art. The active ingredient of the hypoglycemic agent of the present invention, is lipophilic. Therefore, the active ingredient can be dissolved in an oily mediums, for example, natural edible oils such as sesame oil, soybean oil, corn oil, olive oil, cotton seed oil, middle chain fatty acid triglycerides such as Panacete and the like, and then administered orally. For the above administration, an oily solution containing the active ingredient may preferably be encapsulated in capsules and administered orally, and such means are well known to those skilled in the art and ordinarily used.

### Examples

The present invention will be more specifically explained with reference to the following examples. However, the scope of the present invention is not limited to these examples.

### A. Experimental Methods

### (1) Materials for experiments

5-Campesten-3-one was prepared as 24-alkylcholesten-3-one. 5-Campesten-3-one was prepared by the method of Shimizu et al. (Takeshi Shimizu et al., Method for synthesis of steroids: Japanese Patent Unexamined Publication (KOKAI) No.(Hei)11-205889) using campesterol as a starting material (purity not less than 98%, Tama Biochemical Co., Ltd.).

### (2) Experimental animal and condition for bleeding

6-Week old c57BL db/db male mice, as being a Type-2 diabetes model animal having homozygote diabetes pathogenic gene (db), were divided in three groups each consisting of 10 animals. A group of healthy c57BL db/+m male mice (10 animals) having heterozygote db gene was used as a control group. The animals were housed in plastic cages for mice at five animals per cage. The animals were fed under feeding conditions at a temperature of 24±1°C and a relative humidity of 55±5%, where light and darkness were controlled to change every 12 hours, and each cage and bedding materials were exchanged twice a week. The animals were allowed to water ad libitum.

### (3) Experimental feed

Feed CMF for mouse (Oriental Yeast Co.) was used as a basic feed, and 0.1% or 0.3% of 5-campesten-3-one was mixed to the feed to prepare an experimental feed. The resulting feeds were kept at 4°C before the start of feeding.

### (4) Experimental group

The following four experimental groups were arranged and the groups were observed for three months.
First group: Control group 1 (db/+m, healthy mice)
Second group: Control group 2 (db/db, mice with Type-2 diabetes)
Third group: Drug administration group 1 (db/db, the feed supplemented with 0.1% 5-campesten-3-one)
Fourth group: Drug administration group 2 (db/db, the feed supplemented with 0.3% 5-campesten-3-one)

The mice of the first and fourth group were allowed to feed ad libitum, and the mice of the second and third group were fed under the pair feeding method with the amount of feed consistent with that of the fourth group.

### (5) Method for measurement

Measurements of body weights were carried out with passage of time. Bloods were collected from orbital venous plexus every two weeks from the animals, those from 7-week old to 17-week old allowed to feed and water ad libitum, by using a capillary glass in the period of from 10 to 12 a.m., and blood sugar levels were measured by using Glucoase-CII-Test-Wako (Wako Pure Chemicals Industries Co., Ltd.). As for urinary properties, urinary sugar was measured by using a urinary test paper (Uriace-TB, Terumo Co., Ltd.) and one plus (+) or higher results were judged as positive.

For oral glucose tolerance test, the animals, starved for 16 hours from the night before of the end of the feeding (19-week old), were orally administered with glucose (2 g/kg), and the bloods were collected from orbital venous plexus at time 0 and after 60 and 120 minutes by using a capillary glass to measure blood sugar levels.

The animals were observed every day, and after the end of the feeding (19-week old), the animals were anesthetized to death with carbon dioxide gas, and bloods were collected from the abdominal lower vena cava and serums were separated. The animals were autopsied, and the brain, lungs, heart, liver, spleen, pancreas, kidneys, adrenals, and testis were macroscopically examined and weighed.

Triglycerides, total cholesterol, phospholipids, and free fats were measured by using Triglyceride-E-Test Wako, Cholesterol-E-Test Wako, Phospholipid-C-Test Wako, and NEFA-C-Test Wako, respectively. Mean values and standard errors for each groups were calculated on the basis of the values measured, and significant differences were determined by t-test.

### B. Experimental Results,

### (1) Change in blood sugar level

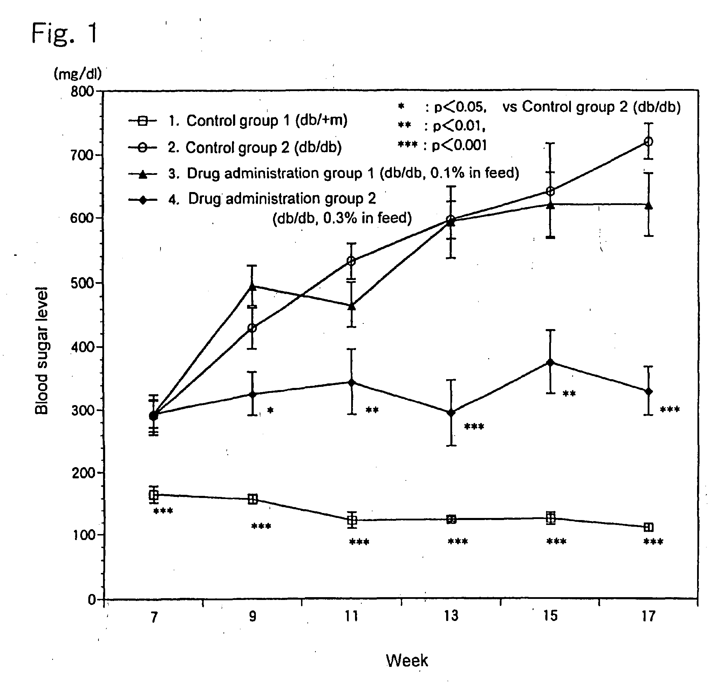

For each groups, changes with time in blood sugar levels are shown in Figure 1. The blood sugar level of the first group consisting of healthy animals was changing in a level within 130 to 160 mg/dl. Whilst, the second group as being model animals of Type-2 diabetes gave linear increase from 270 mg/dl (7-week old) to 720 mg/dl (17 week old). The forth group, as being the model animals of Type-2 diabetes administered with 5-campesten-3-one mixed at 0.3% in the feed, gave a almost constant level without a significant increase from the initial value of 270 mg/dl (7-week old), and also gave significantly lower values at every measuring times after 9-week old as compared to the levels of the control group (the second group). In the third group administered with 5-campesten-3-one mixed at 0.1% in the feed, it was observed that the blood sugar level was increased and decreased in 9-week old and 11-week old, respectively, relative to the control group (the second group), and then became a lower level in 17-week old.

### (2) Oral glucose tolerance test (OGTT)

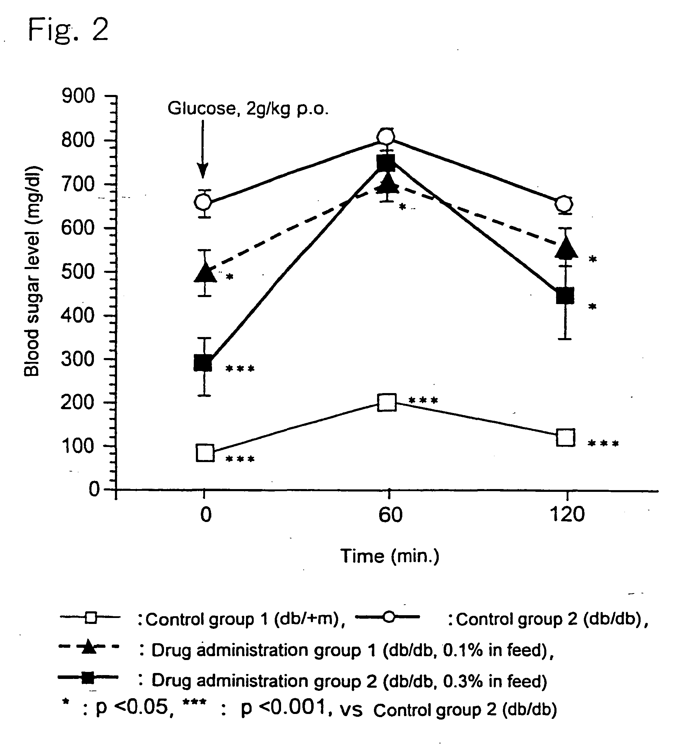

The results of OGTT were shown in Figure 2. The curve of blood sugar level of the second group was 654 mg/dl at time 0, 802 mg/dl at 60 minutes, and 655 mg/dl at 120 minutes, which were remarkably high values as compared to the levels of the healthy mouse (the first group). Whist, the third group gave 497 mg/dl at time 0, 703 mg/dl at 60 minutes, and 558 mg/dl at 120 minutes, which were significantly lower than the levels of the second group at every measuring points. The fourth group gave 282 mg/dl as the value of time 0, which is lower than the half of the level of the second group, and gave almost the same 60-munites value as that of the third group. However, the group gave 440 mg/dl as a 120-minutes value which was lower than that of the third group.

### (3) Urinary sugar

As shown in Table 1, in the group of healthy mice (the first group), all of the mice gave negative results during the test period, except one mouse of 17-week old gave a positive result (10%). Whilst as for urinary sugar of the second group consisting of the mice with Type-2 diabetes, the mice gave a positive ratio of 56% in 7-week old, and all mice gave positive results in 9-week old (100%), which 100% positive rate was maintained during the whole period afterwards. In addition, almost the whole mice in the group gave three plus or more (+++, 2,000 mg/dl) as results of urinary concentrations. As for the urinary sugar in the third group administered with 5-campesten-3-one mixed at 0.1% in the feed, the mice gave a positive ratio of 63% in 7-week old and then 100% in 13-week old, and each of all the mice, a delay in giving a positive result was observed. In the forth group administered with 5-campesten-3-one mixed at 0.3% in the feed, a positive ratio of urinary sugar was found to be increased or decreased in a range of 14 to 70%, however, the group did not give entirely positive results. As for positive degrees of urinary sugar in this group, almost a half of the mice gave one plus (+, 150 mg/dl) or two plus (++, 500 mg/dl), which was lower than the results of the control group (the second group).

**Control group 1 (db/+m)**

| Week | Period | Number of mice | - | ± | + | ++ | +++ | Positive ratio |
|---|---|---|---|---|---|---|---|---|
| | (week) | | | | | | | |
| 7 | 0 | 10 | 10 | 0 | 0 | 0 | 0 | 0 |
| 9 | 2 | 7 | 7 | 0 | 0 | 0 | 0 | 0 |
| 11 | 4 | 10 | 10 | 0 | 0 | 0 | 0 | 0 |
| 13 | 6 | 9 | 9 | 0 | 0 | 0 | 0 | 0 |
| 15 | 8 | 10 | 9 | 1 | 0 | 0 | 0 | 0 |
| 17 | 10 | 10 | 9 | 0 | 1 | 0 | 0 | 10 |

**Control group 2 (db/db)**

| Week | Period | Number of mice | - | ± | + | ++ | +++ | Positive ratio |
|---|---|---|---|---|---|---|---|---|
| | (week) | | | | | | | |
| 7 | 0 | 9 | 4 | 0 | 1 | 1 | 3 | 56 |
| 9 | 2 | 8 | 0 | 0 | 2 | 2 | 4 | 100 |
| 11 | 4 | 10 | 0 | 0 | 0 | 0 | 10 | 100 |
| 13 | 6 | 10 | 0 | 0 | 1 | 0 | 9 | 100 |
| 15 | 8 | 10 | 0 | 0 | 0 | 0 | 10 | 100 |
| 17 | 10 | 10 | 0 | 0 | 0 | 0 | 10 | 100 |

**Drug administration group 1 (db/db, mixed at 0.1% in the feed)**

| Week | Period | Number of mice | - | ± | + | ++ | +++ | Positive ratio |
|---|---|---|---|---|---|---|---|---|
| | (week) | | | | | | | |
| 7 | 0 | 8 | 2 | 1 | 1 | 0 | 4 | 63 |
| 9 | 2 | 7 | 1 | 2 | 0 | 2 | 2 | 57 |
| 11 | 4 | 9 | 1 | 0 | 1 | 0 | 7 | 89 |
| 13 | 6 | 9 | 0 | 0 | 0 | 0 | 9 | 100 |
| 15 | 8 | 9 | 0 | 0 | 0 | 1 | 8 | 100 |
| 17 | 10 | 9 | 0 | 0 | 1 | 0 | 8 | 100 |

**Drug administration group 2 (db/db, mixed at 0.3% in the feed)**

| Week | Period | Number of mice | - | ± | + | ++ | +++ | Positive ratio |
|---|---|---|---|---|---|---|---|---|
| | (week) | | | | | | | |
| 7 | 0 | 7 | 6 | 0 | 0 | 0 | 1 | 14 |
| 9 | 2 | 5 | 4 | 0 | 0 | 1 | 0 | 20 |
| 11 | 4 | 8 | 2 | 1 | 0 | 2 | 3 | 63 |
| 13 | 6 | 10 | 5 | 0 | 1 | 2 | 2 | 50 |
| 15 | 8 | 9 | 4 | 1 | 1 | 1 | 2 | 44 |
| 17 | 10 | 10 | 1 | 2 | 3 | 1 | 3 | 70 |

### (4) Body weight

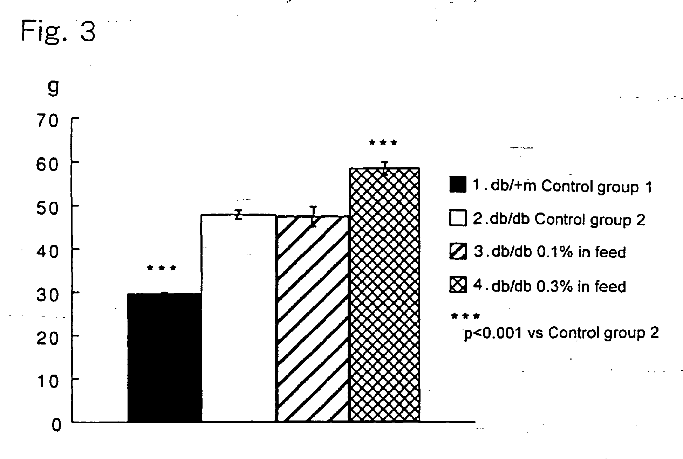

As for the body weights of the mice of 19-week old, obesity was observed in the model mice with Type-2 diabetes as compared to the healthy mice (the first group) as shown in Figure 3. The body weight of the group administered with 5-campesten-3-one mixed at 0.3% in the feed (the forth group) was significantly higher than that of the control group (the second group). The results can be interpreted that, in the same manner of the action of the thiazolidine derivative-type drug as being other drug for treatment of diabetes, the insulin resistance was improved by 5-campesten-3-one, and as a result, an accumulation of body fat by insulin was enhanced.

### (5) Autopsical finding

In autopsy of the brain, heart, lungs, livers, pancreas, spleen, kidneys, and adrenals, no particular abnormality was observed, except that some of the mice with Type-2 diabetes (the second group, the third group, and the forth group) were found to exhibit the conditions of fat liver. The mice with Type-2 diabetes were found to be in copulative impotence, and the weights of their testis were as low as 50 to 60% as compared to that of the healthy mice (the first group). Whilst, the weight of the testis (absolute weight) of the group administered with 5-campesten-3-one mixed at 0.3% in the feed (the forth group) was found to be 85% of that of the healthy mice (the first group).

### (6) General conditions

As for the general conditions of each of the groups during the experimental period, no particular abnormality was observed, except differences in body weights, and no abnormal findings such as diarrhea was observed. A tendency of polyuria was observed in the mice with Type-2 diabetes as compared to the healthy mice (the first group).

### Industrial Applicability

The hypoglycemic agent of the present invention can be used for improvement of hyperglycemia resulting from diseases such as diabetes, and can be used as a safe medicament without side effects such as hypoglycemia or diarrhea.

## Claims

1. Use of 5-campesten-3-one for the preparation of a medicament for the improvement of hyperglycemia.

2. Use according to claim 1, wherein hyperglycemia results from Type 2 diabetes mellitus.

## Patentansprüche

1. Verwendung von 5-Campesten-3-on für die Herstellung eines Medikaments zur Verbesserung von Hyperglykämie.

2. Verwendung nach Anspruch 1, wobei die Hyperglykämie von einem Diabetes mellitus Typ 2 herrührt.

## Revendications

1. Utilisation de 5-campesten-3-one pour la préparation d'un médicament pour l'amélioration de l'hyperglycémie.

2. Utilisation selon la revendication 1, dans laquelle l'hyperglycémie résulte d'un diabète sucré de type 2.
